# EUROPEAN PATENT APPLICATION

(11) **EP 1 464 708 A1**
(43) Date of publication of application: **06.10.2004**
(21) Application number: 03007679.8
(22) Date of filing: 03.04.2003
(51) Int. Cl.: C12P 19/32, C12P 19/40, C07H 19/16, C07H 19/20

(54) **A process for the preparation of fludarabine phosphate from 2-fluoroadenine**

(71) Applicant: Pro. Bio. Sint. S.p.A., 26900 Lodi (IT)
(72) Inventor: Farina, Paolo, 26900 Lodi (IT); Petrucciani, Luigi, 21100 Varese (IT); Colombo, Paolo, 21100 Varese (IT); Caprioli, Giovanni, 21100 Varese (IT)
(74) Representative: Minoja, Fabrizio, Dr.

(57) **Abstract**

The invention provides a process for the preparation of fludarabine phosphate from 2-fluoroadenine and 9-β-D-arabinofuranosyl-uracil using *Enterobacter aerogenes* (EBA).

2-Fluoroadenine is reacted with 9-β-D-arabinosyl-uracile in a water solution at pH = 7 in the presence of EBA cell paste, to yield fludarabine. Fludarabine is then treated with acetic anhydride and the resulting acetylderivative is crystallised and hydrolysed to fludarabine. Phosphorylation and crystallisation afford fludarabine phosphate.

## Description

### FIELD OF THE INVENTION

The present invention relates to a process for the preparation of fludarabine phosphate **(I),** in particular to a process for the preparation of fludarabine phosphate from 2-fluoroadenine and 9-β-D-arabinofuranosyluracil using *Enterobacter aerogenes.*

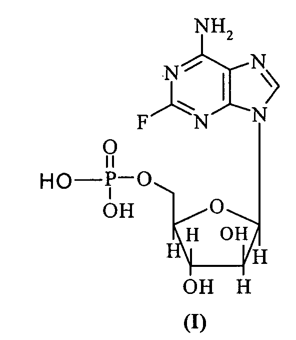

### TECHNOLOGICAL BACKGOUND

Fludarabine (9-β-D-arabinofuranosyl-2-fluoroadenine) **(II)** is a purine nucleoside antimetabolite resistant to adenosine deaminase, employed for the treatment of leukemia.

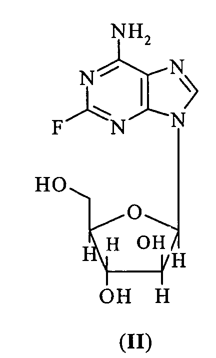

Fludarabine is usually administered as a pro-drug, fludarabine phosphate, which is also the natural metabolite. Fludarabine was firstly synthesised by Montgomery (**US 4,188,378** and **US 4,210,745**) starting from 2-aminoadenine. The method comprised acetylation of 2-aminoadenine, reaction with a benzyl-protected chlorosugar, deacetylation of the amino groups, diazotization and fluorination of the 2-amino group followed by deprotection of the sugar residue.

Fludarabine phosphate can be obtained according to conventional phosphorylation methods, typically by treatment with trimethylphosphate and phosphoryl chloride. Recently, a method for preparing highly pure fludarabine, fludarabine phosphate and salts thereof has been disclosed by Tilstam et al. (**US 6,46,322**).

Enzymatic synthesis has been regarded as a valid alternative to conventional methods for the synthesis of nucleosides and nucleotides derivatives. **EP 0 867 516** discloses a method for the preparation of sugar nucleotides from sugar 1-phosphates and nucleosides monophosphates by use of yeast cells having nucleoside diphosphate-sugar pyrophosphorylase activity. **EP 0721 511 B1** discloses the synthesis of vidarabine phosphate and fludarabine phosphate by reacting an arabinonucleotide with an arylphosphate in the presence of a microorganism able to catalyse the phosphorylation of nucleosides. This method is particularly convenient in that it does not require purified enzymes, but it does not allow to synthesise vidarabine and fludarabine.

### DESCRIPTION OF THE INVENTION

It has now been found that fludarabine can be conveniently prepared by reacting 2-fluoroadenine with 9-β-D-arabinofuranosyl-uracil (Ara-U) in the presence of *Enterobacter aerogenes* (EBA).

The present invention relates to a process for the preparation of fludarabine phosphate **(I)** illustrated in the scheme and comprising the following steps:
a) reaction of 2-fluoroadenine with 9-β-D-arabinofuranosyl-uracil in the presence of *Enterobacter aerogenes* to give crude fludarabine (**II**);
b) treatment of crude fludarabine with acetic anhydride to 2',3',5'-tri-O-acetyl-9-β-D-arabinofuranosyl-2-fluoroadenine (**III**);
c) hydrolysis and recrystallisation of intermediate **(III)** to give pure fludarabine;
d) phosphorylation of fludarabine to give fludarabine phosphate (**I**).

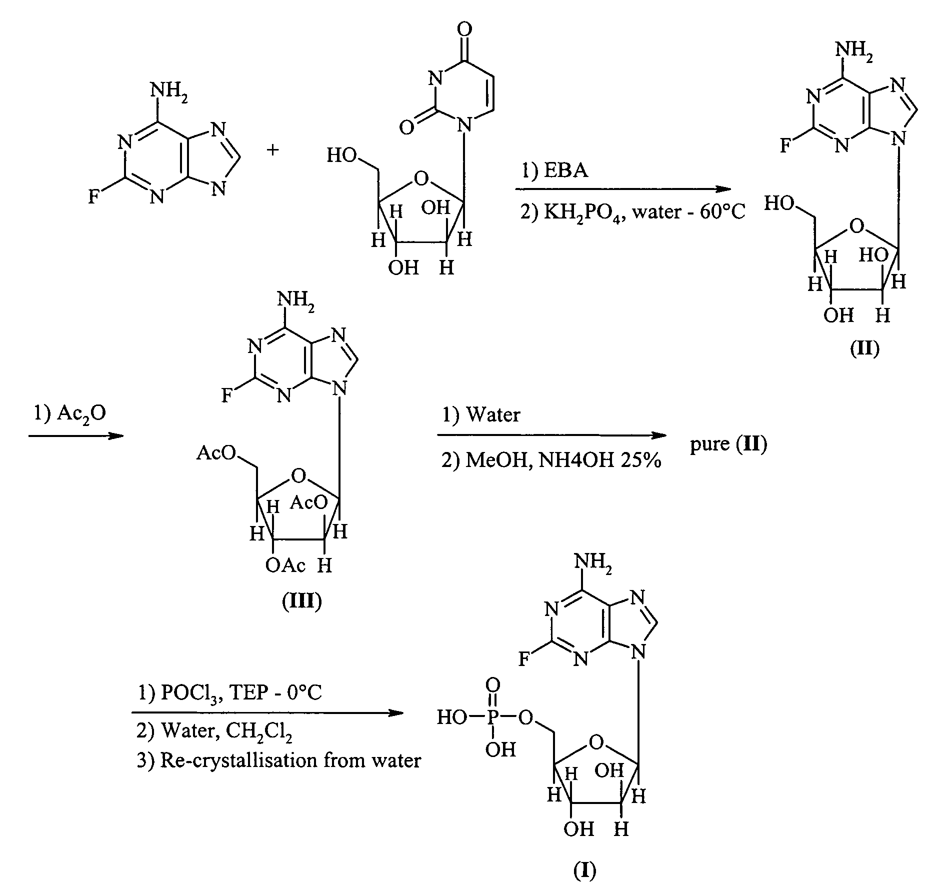

Step a) is carried out in a 0.03 - 0.05 M KH₂PO₄ solution, heated to a temperature comprised between 50 and 70°C, preferably to 60°C, adjusted to pH 7 with KOH pellets and added with 2-fluoroadenine, Ara-U and EBA. The concentration of 2-fluoroadenine in the solution ranges from 0.02 to 0.03 M, while 9-β-D-arabinofuranosyl-uracil is used in a strong excess; preferably, the molar ratio between 9-β-D-arabinofuranosyl-uracil and 2-fluoroadenine ranges from 5:1 to 7:1, more preferably from 5.5:1 to 6.5:1. 2 - 2.5 1 of cell culture per 1 of KH₂PO₄ solution are used. The mixture is stirred at 60°C, adjusting the pH to 7 with a 25% KOH solution and the reaction is monitored by HPLC. Once the reaction is complete (about 24-26 hours), the cell material is separated by conventional dialysis and the permeated solutions are recovered and kept cool overnight. Crystallised fludarabine contains 10% 9-β-D-arabinofuranosyl adenine, which can be conveniently removed by means of steps b) and c).

In step b) crude fludarabine from step a) is dissolved in 9-11 volumes of acetic anhydride, preferably 10 volumes and reacted at 90 - 100°C under stirring, until completion of the reaction (about 10 - 12 h). Acetic anhydride is co-evaporated with acetone and the product is suspended in water.

The hydrolysis of step c) is carried out with methanol and ammonium hydroxide. Typically, compound **(III)** from step b) is suspended in 9-11 volumes of methanol and 2.5 - 3.5 volumes of 25% NH₄OH and stirred at room temperature until complete hydrolysis (about 20 hours; the completion of the reaction can be promoted by mildly warming up the mixture to 30-32°C). Fludarabine precipitates by cooling the mixture to 10°C and is further hot-crystallised with water, preferably with 50 - 70 ml of water per gram of fludarabine or with a water/ethanol mixture (1/1 v/v) using 30 - 40 ml of mixture per gram of fludarabine. Fludarabine is recovered as the monohydrate and has a HPLC purity higher than 99%.

Even though the conversion of fludarabine into fludarabine phosphate (step d) can be carried out according to any conventional technique, for example as disclosed in US 4,357,324, we have found that an accurate control of the reaction and crystallisation temperature allows to minimise product decomposition and significantly improves the yield. According to a preferred embodiment of the invention, the reaction between phosphorus oxychloride, triethylphosphate and fludarabine is carried out at -10°C, and fludarabine phosphate is precipitated from water at 0°C.

In summary, the present invention allows to obtain the following advantages: fludarabine is prepared by enzymatic synthesis without the use of pure enzymes and is therefore particularly suitable for industrial scale; fludarabine is easily recovered and purified from 9-β-D-arabinofuranosyl adenine by acetylation without the need of chromatographic purification, since the triacetyl-derivative precipitates from water with high purity and yield; fludarabine phosphate can be obtained in high yield by controlling the reaction and crystallisation temperature in the phosphorylation step.

The following examples illustrate the invention in more detail.

### EXAMPLES

### Example 1 - Crude 9-β-D-arabinofuranosyl-2-fluoroadenine (II)

A solution of KH₂PO₄ (123 g, 0,9 moles) in water (13 l) was heated to 60°C under stirring and the pH adjusted to 7 with KOH pellets (130 g, 2.32 moles), then added with Ara-U (1451 g, 5.94 moles), 2-fluoroadenine (150 g, 0.98 moles) and EBA (ATCC® n° 13048) cell culture (30 l).

The mixture was stirred at 60°C for 24-26 hours, adjusting the pH to 7 with a 25% KOH solution and monitoring the reaction by HPLC.

After 24-26 hours the cell material was separated by dialysis at 50°-55°C, diluting the mixture with water. The permeated yellow clear solutions were collected, pooled (50 l) and left to stand at 0°-5°C overnight. The resulting crystalline precipitate was filtered and washed with cold water (2 l).

The product was dried at 45°C under vacuum for 16 hours to give 110 g of the crude compound **(II)** which was shown by HPLC to be a mixture of (**I**) (90%) and 9-β-D-arabinofuranosyl adenine (10%).

### Example 2 Pure 9-β-D-arabinofuranosyl-2-fluoroadenine (II)

9-β-D-arabinofuranosyl-2-fluoroadenine **(II)** (30 g, 0,095 moles) was suspended in acetic anhydride (300 ml) and heated to 95°C under stirring.

After 7 hours a clear solution was obtained and left to react at 95°C for further 2-3 hours until the acetylation was completed.

The resulting yellow solution was then concentrated under vacuum at 45°C and the residue was co-evaporated with acetone (2 x 50 ml) and suspended in water (600 ml). The water suspension was cooled to room temperature and left under stirring for 1 hour.

The product was collected by filtration and washed with water (2 x 100 ml) to give 34 g of wet 2',3',5'-tri-O-acetyl-9-β-D-arabinofuranosyl-2-fluoroadenine **(III).**

Wet compound **(III)** was suspended in methanol (300 ml) and added with 25% NH₄OH (100 ml). The mixture was left to stand at room temperature overnight and after 19 hours was warmed to 30°-32°C for 3 hours, until no starting material was detected by HPLC.

The suspension was cooled to 10°C for 1 hour, then the product was collected by filtration and washed with a methanol-water mixture (2 x 25 ml, 3:1 v/v). The product was dried under vacuum at 45°C overnight to give 17.5 g of fludarabine (**II**) (98.4% HPLC purity).

### Method A

Re-crystallisation of compound **(II)** (17.5 g, 0.061 moles) was also carried out by suspending the product in water (875 ml) and heating to 95°C until a clear solution was obtained. The solution was allowed to cool spontaneously to room temperature and the crystalline product was filtered, washed with cold water (2 x 50 ml) and dried under vacuum at 45°C overnight, to give 15.5 g of pure fludarabine **(II)** as the monohydrate (99.3% HPLC purity).

The monohydrate was further dried under vacuum at 90°C for 24 hours to give pure anhydrous fludarabine **(II).**

### Method B

Fludarabine (II) (35 g, 0.123 moles) was also re-crystallized by suspending the product in a water/ethanol mixture (1/1, v/v) (1050 ml) and heating to 80°C until a clear solution was obtained. The solution was allowed to cool spontaneously to room temperature and the crystalline product was filtered, washed with a water/ethanol mixture (2 x 50 ml) and dried under vacuum at 45°C overnight, to give 32 g of pure fludarabine (II) as the monohydrate ( 99% HPLC purity ).

The monohydrate was further dried under vacuum at 90°C for 24 hours to give pure anhydrous fludarabine **(II).**

### Example 3 - 9-β-D-arabinofuranosyl-2-fluoroadenine-5'-phosphate (I)

### Method A

Phosphorous oxychloride (5 g, 3 ml, 0.033 moles) was added to cold (0°C, ice-bath) triethylphosphate (50 ml) and the solution was kept at 0°C for 1 hour, thereafter added with anhydrous fludarabine **(II)** (5 g, 0.018 moles) under stirring.

After about 3 hours, the reaction mixture became homogeneous and turned light-yellow and was kept at 0°C overnight. Once the phosphorylation was completed (about 23 hours) the mixture was added with water (10 ml) and the solution was stirred for 3 hours at 0°C. The mixture was then poured into cold (0°C) methylene chloride (400 ml) and kept at 0°C under stirring until a clear methylene chloride phase was obtained (at least 1 hours).

The methylene chloride phase was removed by decantation and the residual yellowish oil was dissolved in warm (50°C) water (30 ml). The solution was allowed to cool spontaneously to room temperature overnight and the resulting crystalline product was collected by filtration and washed with water (10 ml) and ethanol (2 x 10 ml).

The product was dried at room temperature under vacuum for 24 hours to give 4 g of compound (**I**).

Compound (**I**) was re-crystallised as follows: compound (**I**) (4 g) was dissolved in 60 ml of preheated deionized water (73°-75°C) and the solution was stirred and rapidly cooled to 50°C to minimize product decomposition. The solution was then allowed to cool spontaneously to room temperature: the precipitation started at 40°C. The resulting precipitate was collected by filtration and washed with water (10 ml) and ethanol (2 × 10 ml). The product was dried at room temperature under vacuum for 24 hours to give 2.5 g of compound **(I).**

### Method B

Phosphorous oxychloride (5 g, 3 ml, 0.033 mol) was added to cold (-10°C) triethylphosphate (50 ml) and the solution was kept at -10°C for 1 hour, thereafter anhydrous fludarabine **(II)** (5 g, 0,018 mol) was added with stirring at -10°C.

After about 6 hours the reaction mixture turned light-yellow and became homogeneous. The mixture was kept at -10°C overnight and after 23 hours the phosphorylation was completed. After addition of 40 ml of cold water (2°C) the solution was stirred for 1 hour at 0°C and extracted with cold (0°C) methylene chloride (100 ml and two 50-ml portions).

The aqueous solution was kept under vacuum at room temperature for 1 hour and allowed to stand at 0°C for 24 hours. The resulting crystalline product (**I**) was collected by filtration and washed with ethanol (2 x 20 ml).

The product was dried at 40°C under vacuum for 24 hours (Yield: 5 g).

A final crystallization was carried out as follows. Compound (**I**) (5 g) was dissolved in 75 ml of preheated deionized water (73°-75°C) and the solution was stirred and rapidly cooled to 50°C to minimize decomposition. The solution was then allowed to cool spontaneously to room temperature (the precipitation started at 40°C). The resulting precipitate was collected by filtration and washed with water (10 ml ) and ethanol (2 x 10 ml). The product was dried at 40°C under vacuum for 24 hours (Yield: 4 g).

## Claims

1. A process for the preparation of fludarabine phosphate (**I**) 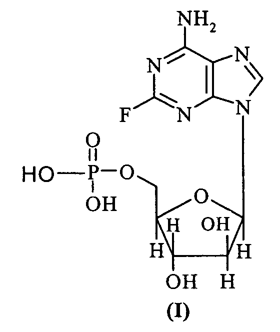 comprising the following steps:
a) reaction of 2-fluoroadenine with 9-β-D-arabinofuranosyl-uracil in the presence of *Enterobacter aerogenes* to give crude fludarabine (**II**); 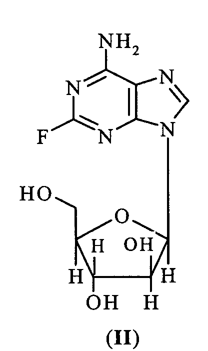
b) treatment of crude fludarabine with acetic anhydride to give 2',3',5'-tri-O-acetyl-9-β-D-arabinofuranosyl-2-fluoroadenine (**III**); 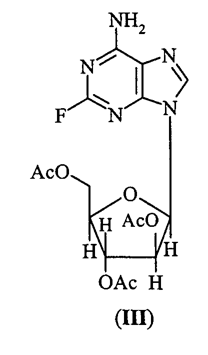
c) hydrolysis and recrystallisation of compound **(III)** to give pure fludarabine **(II);**
d) phosphorylation of fludarabine to give fludarabine phosphate (**I**).

2. A process according to claim 1 wherein step a) is carried out at a temperature comprised between 50 and 70°C, and the molar ratio between 9-β-D-arabinofuranosyl-uracil and 2-fluoroadenine ranges from 5:1 to 7:1.

3. A process according to claim 1 or 2 wherein crude fludarabine from step a) is recovered by dialysis.

4. A process according to anyone of claims 1-3 wherein step b) is carried out by dissolving crude fludarabine in 9-11 volumes of acetic anhydride at 90-100°C.

5. A process according to any one of claims 1-4 wherein intermediate (**III**) from step b) is hydrolysed with methanol and ammonium hydroxide.

6. A process according to any one of claims 1-5 wherein fludarabine obtained from step c) is hot-crystallised from water.

7. A process according to any one of claims 1-6 wherein the phosphorylation reaction of step d) is carried out at -10°C and the resulting fludarabine phosphate is precipitated from water at 0°C.
